# EUROPÄISCHE PATENTSCHRIFT

(11) **EP 1 387 657 B1**
(45) Veröffentlichungstag und Bekanntmachung des Hinweises auf die Patenterteilung: **28.10.2009**
(21) Anmeldenummer: 02753049.2
(22) Anmeldetag: 13.05.2002
(51) Int. Cl.: A61K 6/00, C09J 4/00

(54) **SELBSTADHÄSIVE DENTALMATERIALIEN**
SELF-ADHESIVE DENTAL MATERIALS
MATERIAUX DENTAIRES AUTO-ADHESIFS

(30) Priorität: 16.05.2001 DE 10124028
(43) Veröffentlichungstag der Anmeldung: 11.02.2004
(73) Patentinhaber: 3M ESPE AG, 82229 Seefeld (DE)
(72) Erfinder: HECHT, Reinhold, 86916 Kaufering (DE); LUDSTECK, Manfred, 82538 Geretsried (DE); LUCHTERHANDT, Thomas, 86926 Greifenberg (DE); MIKULLA, Markus, 82346 Andechs-Frieding (DE)
(86) Internationale Anmeldenummer: PCT/EP2002/005218
(87) Internationale Veröffentlichungsnummer: WO 2002/092021

(56) Entgegenhaltungen:
- EP-A- 0 712 622
- WO-A-01/10389

## Beschreibung

Die vorliegende Erfindung betrifft selbstadhäsive Dentalmaterialien, die sich durch hohe Adhäsion an Zahnhartsubstanzen, Einfachheit in der Anwendung sowie durch gute mechanische Eigenschaften auszeichnen. Die erfindungsgemäßen Massen können im Dentalbereich als Füllungen, Zemente, Stumpfaufbauten, Fissurenversiegeler und als zahntechnische Werkstoffe eingesetzt werden.

Nach dem heutigen Stand der Technik werden für die oben genannten Anwendungszwecke im wesentlichen folgende Substanzklassen verwendet:
- Amalgame
- Glasionomerzemente (GIZe)
- Composite
- Compomere
- Kunststoffverstärkte Glasionomerzemente (RMGIZe)

Es ist bekannt, dass Amalgame keine Selbstadhäsion an den Zahnhartsubstanzen Schmelz und Dentin zeigen. Die Befestigung von Amalgamen erfolgt rein mechanisch über eine sogenannte Unterschnittpräparation. Es liegt daher zwischen Zahnhartsubstanz und dem Amalgam ein Randspalt vor, der häufig die Ursache für die unerwünschte Bildung von Sekundärkaries ist. Dieser Sachverhalt wird beispielsweise in der WO 93/12759 ausführlich beschrieben.

Die Glasionomerzemente (GIZe) zeigen im Gegensatz zum Amalgam eine schwache Adhäsion an den Zahnhartsubstanzen. Die Haftwerte liegen jedoch im sehr niedrigen Bereich von 1 MPa und werden in der Regel nur durch einen vorgeschalteten Konditionierschritt erreicht. Ein weiterer Nachteil der GIZe sind die niedrigen mechanischen Werte und hier insbesondere die niedrigen Biegefestigkeiten, die einen Einsatz im Kaudruck-belasteten Bereich limitieren.

Composite zeichnen sich durch sehr gute mechanische Eigenschaften sowie durch eine ausgezeichnete Ästhetik aus. Composite bestehen in der ausgehärteten Form im wesentlichen aus einer vernetzten polymeren Kunststoffmatrix auf Basis von (Meth)acrylatmonomeren und einem Anteil von Füllstoffen. Um die Verbindung zwischen Kunststoffmatrix und Füllstoff vor Zersetzungen durch Hydrolyse zu schützen, sind Composite in der Regel unpolar und hydrophob formuliert. Dies hat zur Folge, dass Composite keine Selbstadhäsion an den Zahnhartsubstanzen und insbesondere an Dentin zeigen. Ferner zeigen Composite während der Aushärtung eine Schrumpfung, was die Bildung von Randspalten und die Entstehung von Sekundärkaries ermöglicht. Um die Composite adhäsiv an den Zahnhartsubstanzen zu befestigen und zur Vermeidung des Randspaltes sind zusätzliche Vorbehandlungen bzw. Arbeitsschritte mit einem sogenannten Bonding erforderlich. Dabei müssen nach dem gegenwärtigen Stand der Technik durch den Behandler folgende Arbeitsschritte durchgeführt werden:
- Anätzen der gesamten Zahnhartsubstanzen durch eine geeignete Säure wie Phosphorsäure ("Total Etch-Technik");
- Auftragen eines Primers, der oberflächlich in die Zahnhartsubstanz einpenetriert;
- Auftragen eines Bondings, welches zusammen mit dem Primer eine Hybridschicht bildet;
- Polymerisation des Bondings beispielsweise durch Bestrahlen mit Licht; und / oder Redoxreaktion;
- Aufbringen des eigentlichen Composites.

Um die Anzahl der Arbeitsschritte zu reduzieren sind mittlerweile folgende individuelle neue Verfahren entwickelt worden:
- Zusammenfassen des Primers und Bondings zu einer Komponente.
- Zusammenfassen des Primers und Ätzmittels zu einer Komponente, welche nach dem Auftragen nicht mehr abgespült wird.
- Zusammenfassen des Ätzmittels, Primers und Bondings zu einer Lösung, welche nur noch aufgetragen und ausgehärtet zu werden braucht.

Die Verarbeitung von Compositen ist also zeitaufwendig und durch deren Empfindlichkeit gegenüber Feuchtigkeit bei der Aushärtung zudem arbeitsintensiv (Legen von Kofferdam). Die genannten Probleme sind beispielsweise in W. Geurtsen, Klinik der Kompositfüllung, Carl Hanser Verlag, München, Wien 1989 dokumentiert.

Compomere sind in ihrer Chemie den Compositen verwandt. Sie sind jedoch durch den Einsatz von säurefunktionellen (Meth)acrylaten in der Monomermischung hydrophiler formuliert. Bei Anwendung dieser Materialien ist im Gegensatz zu den Compositen keine absolute Trockenhaltung mehr erforderlich (kein Legen von Kofferdam). Allerdings erfordern auch diese Materialien zur Erzielung eines guten Haftverbundes mit den Zahnhartsubstanzen die Verwendung eines Bondings.

Die RMGIZe bestehen im wesentlichen aus einem basischen Füllstoff, Säuren, Wasser, Monomeren auf (Meth)acrylatbasis und Initiatoren für eine radikalische Polymerisation. Die RMGIZe härten sowohl über eine Säure / Base-Reaktion als auch über eine radikalische Polymerisation aus. Die RMGIZe zeigen im Vergleich zu den konventionellen GIZen eine verbesserte Ästhetik. Auch hier ist für eine - allenfalls minimale - Haftung an der Zahnhartsubstanz in der Regel ein Konditionierschritt erforderlich. Ferner enthalten die Formulierungen der auf dem Markt befindlichen Produkte Hydroxyethylmethacrylat (HEMA), welches als Lösevermittler für die wasserlöslichen Säuren und restlichen (Meth)acrylate fungiert. HEMA ist aus toxikologischer Sicht bedenklich und beeinflußt das Quellverhalten und damit die mechanischen Eigenschaften wie die Biegefestigkeiten der RMGIZe nachteilig.

Die WO 01/10389 A1 beschreibt die Verwendung eines Adhäsivsystems, das radikalisch polymerisierbar ist und ein reaktives Lösungsmittel enthält, zur Befestigung von Materialien, die nur oder auch kationisch polymerisierbar sind, auf Wasser enthaltendem Hartgewebe.

Eine Aufgabe der vorliegenden Erfindung kann somit darin gesehen werden, Dentalmaterialien zur Verfügung zu stellen, die die beschriebenen Nachteile des Stands der Technik nicht aufweisen und sich insbesondere durch eine einfache Anwendung, hohe Adhäsion an Zahnhartsubstanzen ohne Vorbehandlung wie z. B. durch Einsatz eines Konditionierers, Bondings, Primers oder Ätzmittels sowie guten mechanischen Eigenschaften auszeichnen.

Überraschenderweise wurde gefunden, dass sich die erfindungsgemäße Aufgabe durch Formulierungen, die folgende Komponenten umfassen, lösen läßt:
(A) 5 bis 75 Gew.-% einer oder mehrerer mono- oder höherfunktioneller ethylenisch ungesättigter Verbindungen, die zusätzlich über mindestens eine säurefunktionelle Gruppe verfügen, wobei mindestens eine der Verbindungen mindestens eine P-OH-Gruppe aufweist, wie z. B. eine Phosphor-, Phosphon- oder Phosphinsäuregruppe,
(B) 2 bis 50 Gew.-% einer oder mehrerer mono- oder höherfuktioneller ethylenisch ungesättigter Verbindungen ohne säurefunktionelle Gruppe,
(C) 22,8 bis 85 Gew.-% Füllstoff(e), umfassend mindestens einen Füllstoff, der mit der Komponente (A) im Sinne einer Ionenaustausch-, Neutralisations-, Salzbildungs- und/oder Chelatbildungsreaktion zu reagieren vermag,
(D)0,1 bis 8 Gew.-% eines oder mehrerer Initiatoren und gegebenenfalls Aktivatoren,
(E) 0,1 bis 20 Gew.-% zusätzliche Additive bzw. Modifikatoren,
wobei das Gewichtsverhältnis in % von Komponente (A) zu Komponente (B) im Bereich von 21 bis 90 : 10 bis 79, bevorzugt im Bereich von 25 bis 90 : 10 bis 75, besonders bevorzugt 30 bis 90 : 10 bis 70 und ganz besonders bevorzugt 40 bis 80 : 20 bis 60 liegt, und wobei das Wasseraufnahmevermögen bestimmt gemäß EN ISO 4049 : 2000 kleiner 40 µg/mm³ beträgt.

Es hat sich gezeigt, dass Formulierungen mit obiger Zusammensetzung auf Rinderzahnschmelz oder -dentin einen Haftwert von mindestens 2,0 MPa, vorzugsweise von mindestens 2,5 MPa, besonders bevorzugt mindestens 3,0 MPa aufweisen, gemessen gemäß der unten angegebenen Haftwertbestimmungsmethode "Bestimmung der Haftung", ohne dass die Zahnhartsubstanz vorbehandelt zu werden braucht. Die erfindungsgemäßen Formulierungen weisen zudem gute mechanische Eigenschaften auf und sind einfach zu handhaben.

Folgende Eigenschaften sind kennzeichnend für die erfindungsgemäßen Formulierungen:
Haftwert von mindestens 2,0 MPa, vorzugsweise mindestens 2,5 MPa, besonders bevorzugt mindestens 3,0 MPa geringes Wasseraufnahmevermögen von < 40 µg / mm³, bevorzugt < 30 µg / mm³
Biegefestigkeit von > 30 MPa, bevorzugt > 40 MPa

Unter den Begriff "vorbehandeln" fallen Schritte wie z. B. Ätzen, Primen, Bonden, Konditionieren.

Mit den Begriffen "umfassen", "aufweisen" oder "enthalten" wird eine nicht abschließende Aufzählung von Merkmalen eingeleitet. Gleichermaßen ist der Begriff "ein" im Sinne von "mindestens ein" zu verstehen.

Mindestens ein Monomer der Komponente (A) weist mindestens eine P-OH-Gruppe auf, wie z. B. eine Phosphor-, Phosphon- oder Phosphinsäuregruppe. Dieses Monomer liegt dabei vorzugsweise in einer Konzentration von mindestens etwa 5 Gew.-%, besonders bevorzugt von mindestens etwa 10 Gew.-% vor bezogen auf die Bestandteile (A) bis (E).

Bezogen auf den Anteil innerhalb der Komponente (A) liegt dieses Monomer vorzugsweise in einem Gehalt von mindestens 30, bevorzugt mindestens 50 Gew.-% vor.

Es hat sich auch gezeigt, dass eine Formulierung, bei der das Gewichtsverhältnis (in %) von Komponente (A) zu Komponente (B) im Bereich von 21 bis 90 : 10 bis 79, vorzugsweise im Bereich von 25 bis 90 : 10 bis 75, besonders bevorzugt 30 bis 90 : 10 bis 70 und ganz besonders bevorzugt 40 bis 80 : 20 bis 60 liegt besonders vorteilhafte Eigenschaften aufweist.

Bei der Komponente (A) handelt es sich um Verbindungen, die über mindestens eine ethylenisch ungesättigte Gruppierung sowie mindestens eine säurefunktionelle Gruppe verfügen. Bei den polymerisierbaren Gruppierungen handelt es sich um Acryl-, Methacryl-, Vinyl- und/oder Styrylgruppen, wobei Acryl- und Methacrylgruppen besonders bevorzugt sind.

Geeignete Säuregruppen sind beispielsweise Carbonsäurereste, Säurereste des Phosphors (z.B. Phosphor-, Phosphon-, Phosphinsäuren), Schwefels (z.B. Schwefel, Sulfon-, Sulfinsäuren) und des Bors. Die Säuregruppen zeichnen sich dadurch aus, dass sie mit reaktiven anorganischen Füllstoffen lonenaustausch-, Neutralisations-, Salzbildungs- und/oder Chelatbildungsreaktionen eingehen können. Es ist auch möglich, dass die Säurereste der Komponente (A) nicht vollständig in freier, sondern auch zum Teil in derivatisierter Form wie als Salz, Säurehalogenid, Säureanhydrid oder leicht hydrolysierbare Ester vorliegen.

Geeignete Komponenten (A) sowie deren Herstellung werden beispielsweise in der DE 35 36 076 A1, EP 0 237 233 A und WO 95/22956 beschrieben. Exemplarisch seien genannt: 4-(Meth)acryloyloxyethyltrimellithsäure, Butentricarbonsäure, Bis-4,6- bzw. Bis-2,5-(Meth)acryloyloxyethyltrimellithsäure, Phosphorsäureester von Hydroxyethyl(meth)acrylat (HEMA), Glycerindi(meth)acrylat und/oder Pentaerythrittri(meth)acrylat, Chlor- und Bromphosphorsäureester des Bisphenol-A-Glycidyl-(meth)acrylats. Auch führt die Verwendung von Oligomeren oder polymeren Derivaten oben genannter Verbindungen zu erfindungsgemäßen Dentalmaterialien mit guten Eigenschaften.

Besonders bevorzugt sind die Umsetzungsprodukte von nucleophilen Acrylaten und Methacrylaten wie z. B. 2-Hydroxyethylmethacrylat (HEMA) oder Glycerinmethacrylatestern mit reaktiven Phosphonsäure- oder Phosphinsäure- oder Phosphorsäurederivaten wie z. B. POCl₃, P₂O₅ oder PCl₃.

Bevorzugterweise weisen die oben genannten ethylenisch ungesättigten Säuren und/oder deren Derivate ein Molekulargewicht im Bereich von 70 bis 5000, vorzugsweise im Bereich von 90 bis 2500, besonders bevorzugt im Bereich von 100 bis 1000 g/mol auf.

Als Komponente (B) eignen sich Verbindungen, die über mindestens eine ethylenisch ungesättigte Gruppierung verfügen. Bei den polymerisierbaren Gruppierungen handelt es sich um Acryl-, Methacryl-, Vinyl- und / oder Styrylgruppen, wobei Acryl- und Methacrylgruppen besonders bevorzugt sind.

Geeignete mono- und höherfunktionelle (Meth)acrylate sowie weitere ethylenisch ungesättigte Verbindungen werden beispielsweise in der EP 0 480 472 A, der DE 39 41 629 C2 und in G.Webster (Ed.), Chemistry & Technology of UV & EB Formulation for Coatings,lnks and Paints, Vol II Prepolymers & Reactive Diluents, J. Wiley and Sons, Chichester, New York, Weinheim, Brisbane, Toronto, Singapore, 1997 beschrieben. Die ethylenisch ungesättigten Verbindungen können in alleiniger Form oder in Mischungen in den Formulierungen eingesetzt werden.

Geeignete Monomere sind beispielsweise die Acrylsäure- und Methacrylsäureester mono-, di- oder höherfunktioneller Alkohole. Exemplarisch seien genannt: Methyl-(meth)acrylat, Butyl(meth)acrylat, 2-Ethylhexyl(meth)acrylat, Lauryl(meth)acrylat, 2-Hydroxyethyl(meth)acrylat, 2-Hydroxypropyl(meth)acrylat, Glycerin-1,3-di(meth)-acrylat (GDMA), Glycerin-1,2-di(meth)acrylat, Cyclohexyl(meth)acrylat, Phenyl-(meth)acrylat, Isobornyl(meth)acrylat, Ethylenglykoldi(meth)acrylat, 1,4-Butandioldi(meth)acrylat, Triethylenglykoldi(meth)acrylat (TEGDMA), 1,6-Hexandioldi(meth)acrylat, 1,12-Dodecandioldi(meth)acrylat,Trimethylolpropantri(meth)acrylat, Pentaerythrittetra(meth)acrylat und Dipentaerythrithexa(meth)acrylat.

Vorteilhaft einsetzbar sind weiterhin Bisphenol-A-di(meth)acrylat sowie die davon abgeleiteten ethoxy-bzw. propoxylierten Di(meth)acrylate. Auch die in der US 3,066,112 A beschriebenen Monomere auf Basis von Bisphenol-A und Glycidyl-(meth)acrylat oder deren durch Addition von Isocyanaten entstandenen Derivate sind geeignet.

Gut geeignet sind weiterhin die in der DE 28 168 23 C genannten Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und / oder Propylenoxideinheiten verlängerten Verbindungen des Bis(hydroxymethyl)-tricyclo[5.2.1.0^{2,6}]-decans.

Auch Urethan(meth)acrylate wie 7,7,9-Trimethyl-4,13-dioxo-5,12-diazahexadecan-1,16-dioxy-di(meth)acrylat (UDMA, Plex 6661) können Bestandteil der Komponente (B) sein. Bevorzugterweise weisen die genannten Verbindungen und deren Derivate ein Molekulargewicht im Bereich von 70 bis 5000, vorzugsweise im Bereich von 90 bis 2500, besonders bevorzugt im Bereich von 100 bis 1000 g/mol auf.

Als Komponente (C) können anorganische Füllstoffe wie z. B. Gläser oder Keramiken und/oder organische Füllstoffe verwendet werden. Die Füllstoffe können in alleiniger Form bzw. in Mischungen eingesetzt werden. Ferner können zur Optimierung der Produkteigenschaften die Füllstoffe in unterschiedlichen Korngrößen in die Rezepturen eingebracht werden, d.h. die Füllstoffe können eine unimodale oder polymodale, beispielsweise bimodale Verteilung aufweisen.

Es muss mindestens ein Füllstoff eingesetzt werden, der mit der Komponente (A) im Sinne einer lonenaustausch-, Neutralisations-, Salzbildungs- und / oder Chelatbildungsreaktion zu reagieren vermag (reaktiver Füllstoff). Zusätzlich können solche Füllstoffe eingesetzt werden, die gegenüber den Säurefunktionen der Komponente (A) inert sind (nichtreaktive Füllstoffe). Ferner können auch verstärkend wirkende Materialien, wie Fasern oder faserige Verbindungen zugesetzt werden.

Füllstoffe, die mit den Säuregruppen der Komponente (A) zu reagieren vermögen, werden z.B. zur Herstellung von Polycarboxylat- und Glasionomerzementen verwendet und sind z.B. in D.C. Smith, Biomaterials 19, 467-478 (1998), der DE 20 61 513 A und der WO 95/22956 beschrieben.

Prinzipiell geeignet sind feinverteilte Metalle wie feinverteiltes Zink, Metallverbindungen wie die Oxide bzw. Hydroxide von Calcium, Magnesium, Strontium und Zink. Weiterhin geeignet sind basische Glaspulver mit einem hohen Anteil an 2- und 3-wertigen Ionen sowie Metallkationen-freisetzende Silikate wie Schichtsilikate, Bentonite oder Calciumsilikate, Natriumaluminiumsilikate und Zeolithe einschließlich der Molekularsiebe, sowie Apatit. Ebenfalls geeignet als gegenüber der Komponente (A) reaktive Gläser sind die in der WO 93/12759 genannten Borat-, Phosphat- und Fluoroaluminosilikatgläser. Besonders bevorzugte reaktive Füllstoffe sind die Fluoroaluminosilikatgläser sowie Hydroxide der Erdalkalimetalle.

Als inerte anorganische Füllstoffe sind z.B. Quarz, Zirkonsilikate, Fällungskieselsäuren (HDKH) und schwerlösliche Metallsalze wie Bariumsulfat oder Calciumfluorid geeignet. Weitere inerte anorganische Füllstoffe werden in der WO 95/22956 beschrieben. Besonders bevorzugte inerte anorganische Füllstoffe sind Quarz und Zirkonsilikate. Die Komponente (C) umfasst im Sinne der Erfindung hierbei keine pyrogenen Kieselsäuren.

Als organische Füllstoffe sind beispielhaft perlförmige Polymere und Copolymere auf Basis von Methylmethacrylat, die kommerziell unter der Bezeichnung "Plexidon" oder "Plex" von der Firma Röhm erhältlich sind, zu nennen. Besonders geeignet sind auch die in der DE 19 941 738 beschriebenen organischen Füllstoffe auf Polyurethanbasis.

Zum besseren Einbau in die Polymermatrix kann es von Vorteil sein, die genannten Füllstoffe sowie gegebenenfalls röntgenopake Zusatzstoffe mit für den Fachmann bekannten Verfahren oberflächlich zu behandeln bzw. zu beschichten. Beispielhaft genannt sei die Oberflächenbehandlung mit einem Silan wie dem Methacryloxypropyltrimethoxysilan. Die Menge an eingesetzten Beschichtungsmittel liegt üblicherweise zwischen 0,05 und 10 Gew.-%, bevorzugt zwischen 0,1 und 5 Gew.-%, bezogen auf den Füllstoff.

Unter Initiatoren gemäß Komponente (D) sind Initiatorsysteme, die die radikalische Polymerisation der Monomeren bewirken, beispielsweise Photoinitiatoren und/oder sogenannte Redoxinitiatorsysteme und/oder thermische Initiatoren zu verstehen.

Geeignete Photoinitiatoren sind beispielsweise in J.-P Fouassier, Photoinitiation,Photopolymerization and Photocuring, Hanser Publishers, Munich,Vienna, New York 1995 oder auch J.F. Rabek (Hrsg.), Radiation Curing in Polymer Science and Technology, Vol. II, Elsevier Applied Science, London, New York, 1993 sowie in den Patentveröffentlichungsschriften EP 0.073 413 A, EP 0 007 508 A, EP 0 047 902 A, EP 0 057 474 A und EP 0 184 095 A beschrieben. Exemplarisch seien genannt Benzoinalkylether, Benzilketale und Acylphosphinoxide. Besonders geeignet sind aliphatische und aromatische 1,2-Diketonverbindungen wie Campherchinon in Kombination mit Aktivatoren wie tertiären Aminen.

Als Redoxinitiatorsysteme eignen sich beispielsweise organische oxidativ wirksame Verbindungen, wie Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoyl- und p-Methylbenzoylperoxid in Betracht.

Als Aktivatoren eignen sich beispielsweise tertiäre aromatische Amine, wie die aus der US 3,541,068 A bekannten N,N-Bis-(hydroxyalkyl)-3,5-xylidine sowie die aus der DE 26 58 538 A bekannten N,N-bis-(hydroxyalkyl)-3,5-di-t-butylaniline, insbesondere N,N-Bis-(β-oxybutyl)-3,5-di-t-butylanilin sowie N,N-Bis(hydroxyalkyl)-3,4,5-trimethylanilin. Gut geeignete Aktivatoren sind auch Schwefelverbindungen in der Oxidationsstufe +2 oder +4 wie Natriumbenzolsulfinat oder Natriumparatoluolsulfinat oder die in der DE 14 955 20 B beschriebenen Barbitursäuren bzw. Barbitursäurederivate sowie die in der EP 0 059 451 A beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung der Aushärtung kann die Polymerisation in Gegenwart von Schwermetallverbindungen auf Basis von z.B. Ce, Fe, Cu, Mn, Co, Sn oder Zn durchgeführt werden, wobei Kupferverbindungen besonders geeignet sind. Die Schwermetallverbindungen werden bevorzugt in Form löslicher organischer Verbindungen eingesetzt.

Ein besonders geeignetes Redoxsystem umfasst folgende Komponenten:
I. 14,9 bis 50 Gew.-%, bevorzugt 20 bis 45 Gew.-% einer Barbitursäure oder Thiobarbitursäure bzw. einem Barbitursäure- oder Thiobarbitursäurederivat,
II. 30 bis 75 Gew.-%, bevorzugt 35 bis 67,8 Gew.-% einer Peroxodisulfatverbindung und/oder Peroxodiphosphatverbindung,
III. 10 bis 35 Gew.-%, bevorzugt 12 bis 30 Gew.-% einer Sulfinsäureverbindung und
IV. 0,1 bis 5 Gew.-%, bevorzugt 0,2 bis 4 Gew.-% einer Kupferverbindung.

Werden die erfindungsgemäßen Dentalmassen durch Photopolymerisation ausgehärtet, so können einkomponentige Systeme formuliert werden. Enthalten die erfindungsgemäßen Dentalmassen ein Redoxinitiatorsystem, umfassend z.B. organisches Peroxid und Aktivator, so sind aus Gründen der Lagerstabilität Peroxid und Aktivator in räumlich voneinander getrennten Teilen der erfindungsgemäßen Dentalmasse vorhanden, die erst unmittelbar vor der Anwendung miteinander gemischt werden. Es handelt sich hierbei also um mindestens zweikomponentige Formulierungen in Form von z. B. Pulver/Flüssigkeit oder Paste/Paste.

Aus Gründen der Lagerstabilität können die Bestandteile des erfindungsgemäßen Initiatorsystems mikroverkapselt werden. Verfahren zur Mikroverkapselung sind beispielsweise in der US 5 154 762 und EP 0 588 878 B1 beschrieben.

Zur Einstellung spezieller Eigenschaften können als Komponente (E) zusätzliche Additive oder Modifikatoren in die Formulierungen eingebracht werden. Mögliche Additive und deren Aufgaben werden in U. Zorll (Hrsg.), Lehrbuch der Lacktechnologie, Vincentz Verlag, Hannover 1998 und P. Nanetti, Lackrohstoffkunde, Vincentz Verlag, Hannover 1997 beschrieben. Ohne Anspruch auf Vollständigkeit seien stellvertretend einige Additive bzw. Modifikatoren genannt:
Weichmacher wie Phthalate, Adipate, Sebacate, Phosphorsäureester oder Zitronensäureester, um z.B. die Flexibilität der Massen zu erhöhen;
anorganische und organische Pigmente bzw. Farbstoffe wie Weißpigmente auf Basis von Titandioxid oder Zinksulfid (Lithopone), Eisenoxid Rot 3395, Bayferrox 920 Z Gelb, Neozaponblau 807 (Farbstoff auf Basis Kupfer-Phthalocyanin) oder Helio Echtgelb ER zur individuellen Farbgebung der dentalen Massen;
Stabilisatoren, insbesondere Radikalfänger, wie substituierte und nichtsubstituierte Hydroxyaromaten (z.B. para-Methoxyphenol), Phenothiazin, sogenannte HALS (Hindered Amine Light Stabilizers) und/oder Schwermetallfänger wie EDTA;
Thixotropiehilfsmittel wie pyrogene Kieselsäuren (Aerosil) oder auch modifizierte Schichtsilikate;
ionenabgebende Substanzen, insbesondere solche die Fluoridionen freisetzen wie die Fluoridsalze der Metalle der ersten und / oder zweiten Hauptgruppe wie Natriumfluorid. Besonders geeignet sind komplexe anorganische Fluoride der allgemeinen Formel AₙMFₘ wie in der EP 0 717977 A beschrieben. Dabei bedeutet A ein ein- oder mehrwertiges Kation, M ein Metall der III, IV, V Haupt- oder Nebengruppe, n eine ganze Zahl von 1 bis 3 und m eine ganze Zahl von 3 bis 6. Stellvertretend genannt seien Kaliumzinkfluorid und Kaliumhexafluorotitanat.

Bakterizid oder antibiotisch wirksame Substanzen wie z. B. Chlorhexidin, Pyridiniumsalze oder die üblichen pharmazeutischen Substanzen wie β-Lactamantibiotika (Penicilline), Cephalosporine, Tetracycline, Chloramphenicol, Fosfomycin, antibakterielle Makrolide oder Polypeptid-Antibiotika können ebenfalls eingesetzt werden.

Es können auch Lösungsmittel als Fließmittelverbesserer und zur Verbesserung des Mischungsverhaltens eingesetzt werden. Besonders bevorzugt sind dabei Wasser, Aceton, Methylethylketon und/oder kurzkettige Alkohole mit weniger als 10 Kohlenstoffatomen wie z. B. Ethanol oder iso-Propanol.

Ferner können zur Einstellung spezieller Eigenschaften als Komponente (E) auch lösliche organische Polymere wie Polyvinylacetat, Polyacrylsäure und/oder Polyvinylether eingesetzt werden.

Die erfindungsgemäßen Massen können im Dentalbereich beispielsweise als Füllungsmaterialien, Fissurenversiegeler, Zement, Stumpfaufbauten sowie als zahntechnische Werkstoffe verwendet werden.

Im folgenden wird die Erfindung anhand von Beispielen näher beschrieben, wobei diese als die Erfindung in keinster Weise limitierend zu verstehen sind.

### Experimenteller Teil:

### Beispiel 1: Lichthärtendes Füllungsmaterial als 2 K-System (Pulver / Flüssigkeit), umfassend 3,8 Teile Pulver und 1 Teil Flüssigkeit

### Pulver:

(C) 98 Gew.-% Strontiumaluminiumfluorosilikatglas, silanisiert mit 0,075% Gew.-% Methacryloxypropyltrimethoxysilan
(E) 1 Gew.-% pyrogene Kieselsäure (Aerosil OX 50), silanisiert mit 3 Gew.-% Methacryloxypropyltrimethoxysilan
(C) 1 Gew.-% Calciumhydroxid

### Flüssigkeit:

(A) 49,44 Gew.-% Hydroxyethylmethacrylatphosphat
(B) 30 Gew.-% propoxyliertes Bisphenol A-dimethacrylat
(B) 20 Gew.-% Triethylenglykoldimethacrylat (TEGDMA)
(D) 0,26 Gew.-% Dimethylaminoethylbenzoat
(D) 0,2 Gew.-% Campherchinon
(D) 0,1 Gew.-% Cu(II)-Acetat

### Beispiel 2: Lichthärtendes Füllungsmaterial als 2 K-System (Pulver / Flüssigkeit), umfassend 3,8 Teile Pulver und 1 Teil Flüssigkeit

### Pulver:

(C) 93,5 Gew.-% Strontiumaluminiumfluorosilikatglas silanisiert mit 0,075 Gew.-% Methacryloxypropyltrimethoxysilan
(C) 1 Gew.-% Calciumhydroxid
(D) 0,5 Gew.-% Dimethylaminoethylbenzoat
(E) 5,0 Gew.-% pyrogene Kieselsäure (Aerosil OX 50) silanisiert mit 3 Gew.-% Methacryloxypropyltrimethoxysilan

### Flüssigkeit:

(A) 69,7 Gew.-% 1,3-Glycerindimethacrylatphosphat
(B) 30 Gew.-% TEGDMA
(D) 0,2 Gew.-% Campherchinon
(D) 0,1 Gew.-% Cu(II)-ethylhexanoat

### Beispiel 3: Lichthärtendes Füllungsmaterial als 1 K-Paste

Aus den nachfolgenden Bestandteilen wurde mit Hilfe eines Laborkneters eine Paste hergestellt.
(C) 74 Gew.-% Strontiumaluminiumfluorosilikatglas, silanisiert mit 0,3 Gew.-% Methacryloxypropyltrimethoxysilan
(E) 5 Gew.-% pyrogene Kieselsäure (Aerosil OX 50), silanisiert mit 3 Gew.-% Methacryloxypropyltrimethoxysilan
(C) 1 Gew.-% Calciumhydroxid
(D) 0,4 Gew.-% Dimethylaminoethylbenzoat
(A) 13,46 Gew.-% 1,3-Glycerindimethacrylatphosphat
(B) 6 Gew.-% TEGDMA
(D) 0,04 Gew.-% Campherchinon
(D) 0,1 Gew.-% Cu(II)-Acetat

### Beispiel 4: Dualhärtender Befestigungszement als 2K-System (Pulver / Flüssigkeit), umfassend 3 Teile Pulver und 1 Teil Flüssigkeit

### Pulver:

(C) 88,6 Gew.-% Strontiumaluminiumfluorosilikatglas silanisiert mit 0,3 Gew.-% Methacryloxypropyltrimethoxysilan
(C) 1,6 Gew.-% Calciumhydroxid
(D) 0,8 Gew.-% Natriumtoluolsulfinat
(D) 1,2 Gew.-% 1,3-Dimethyl-5-phenylbarbitursäure
(D) 2,4 Gew.-% Natriumperoxodisulfat
(E) 5,4 Gew.-% pyrogene Kieselsäure (Aerosil OX 50) silanisiert mit 3 Gew. % Methacryloxypropyltrimethoxysilan

### Flüssigkeit:

(A) 49,5 Gew.-% 1,3-Glycerindimethacrylatphosphat
(B) 20 Gew.-% propoxyliertes Bisphenol A-dimethacrylat
(B) 30 Gew.-% Triethylenglykoldimethacrylat
(D) 0,2 Gew.-% Campherchinon
(D) 0,1 Gew.-% Cu(II)-Acetat
(E) 0,2 Gew.-% 2,6-Ditertbutyl-4-methylphenol

### Vergleichsbeispiele (siehe Tabelle 1):

### Kommerziell erhältliche Füllungsmaterialien verschiedener Materialklassen

Ketac Molar (GIZ von 3M ESPE AG)
Fuji II LC (RMGIZ von GC)
Dyract AP (Compomer von Dentsply)
Tetric Ceram (Composite von Vivadent)

### Vergleichsbeispiele (siehe Tabelle 2):

### Kommerziell erhältliche Befestigungszemente verschiedener Materialklassen

Ketac Cem (GIZ von 3M ESPE AG)
Fuji Plus (RMGIZ von GC)
Dyract Cem Plus (Compomer von Dentsply)
Panavia 21 (Composite von Kuraray)

### Beschreibung der durchgeführten Messungen

### Bestimmung der Haftung:

Zur Durchführung von Haftversuchen werden Rinderzähne verwendet. Pro Versuch werden fünf nach dem Extrahieren tiefgefrorene Rinderzähne aufgetaut, von restlichem Zahnfleisch gereinigt und die Wurzel durch Absägen mit einer Diamantsäge abgetrennt. Die noch verbleibende Pulpa wird mit Hilfe einer Pulpanadel entfernt und die Zähne dann mit Leitungswasser gespült. Planes Dentin wird durch labiales Schleifen der Zähne an einer wassergekühlten Diamantschleifscheibe erhalten. Die Zähne werden dann so in Silikon eingebettet, dass die abgeschliffene, gut feucht gehaltene Oberfläche nach oben zeigt und anschließend mit einem feinen Siliziumcarbidschleifpapier nass nachbearbeitet. Dann wird auf jeden Zahn ein Wachsplättchen aufgeklebt, welches eine runde Ausstanzung von 6 mm Durchmesser hat (Prüffeld). Dieses Prüffeld wird mit dem nach Angaben des Herstellers gemischten Material plan gefüllt und nach den Angaben des Herstellers 10 bis 40 Sekunden mit Elipar II (600-800 mW/cm²) ausgehärtet. Autopolymerisierende Materialien werden 1 h bei 36°C und 100% rel. Feuchte gehärtet. Nach der Aushärtung wird das Wachsplättchen entfernt, eine Schraube im rechten Winkel zur Zahnoberfläche auf die überstehende Füllung aufgeklebt und nach einer Lagerung von einem Tag bei 36°C und 100% rel. Feuchte im Abzugsversuch die Haftung an einer Zwick UPM 1455 mit einer Abzugsgeschwindigkeit von 1 mm/min gemessen.

Die Bestimmung der Biegefestigkeit erfolgte gemäß EN ISO 4049 : 2000 (3-Punktbiegeversuch). Die Bestimmung der Wasseraufnahme erfolgte an genormten Prüfkörpern gemäß EN ISO 4049 : 2000.

Die Ergebnisse der Biegefestigkeits- und Haftmessungen sowie der Wasseraufnahme sind in den Tabellen 1 und 2 zusammengestellt (lc = licht gehärtet, dc = dunkel gehärtet).

**Tabelle 1**

| Material | Biegefestigkeit [MPa] | Haftung Dentin [MPa] | Wasseraufnahme [µg/mm³] |
|---|---|---|---|
| Ketac Molar (GIZ), dc | 40 | 0,0 | 61 |
| Fuji II LC (RMGIZ), lc | 43 | 0,4 | 129 |
| Dyract AP (Compomer), lc | 100 | 0,0 | 18 |
| Tetric Ceram (Composite), lc | 108 | 0,0 | 13 |
| Beispiel 1, lc | 70 | 3,4 | 20 |
| Beispiel 2, lc | 72 | 3,7 | 23 |
| Beispiel 3, lc | 75 | 3,3 | 19 |

Die erfindungsgemäßen Beispielrezepturen 1 bis 3 zeigen ohne Vorbehandlung eine deutlich höhere Haftung auf Rinderdentin als alle anderen Materialien. Im Vergleich zu den Glasionomerzementen und kunststoffverstärkten Glasionomerzementen sind zudem die Biegefestigkeiten deutlich erhöht. Die Wasseraufnahmen liegen im Bereich der Compomere und Composite und sind deutlich niedriger als bei den Glasionomerzementen und kunststoffverstärkten Glasionomerzementen.

**Tabelle 2**

| Material | | Biegefestigkeit [MPa] | Haftung Dentin [MPa] | Wasseraufnahme [µg/mm³] |
|---|---|---|---|---|
| Ketac Cem (GIC), dc | | 17 | 0 | 60 |
| Fuji Plus (RMGIC), dc | | 18 | 0,9 | 171 |
| Dyract Cem Plus (Compomer), dc | | 58 | 0 | 51 |
| Panavia 21 (Composite), dc | | 94 | 0 | 28 |
| Beispiel 4 | dc | 61 | 4,1 | 25 |
| | lc | 66 | 5,7 | 24 |

Die erfindungsgemäße Beispielrezeptur 4 zeigt ohne Vorbehandlung eine sehr hohe Haftung auf Rinderdentin. Die Wasseraufnahmen liegen im Bereich der Composite und sind deutlich niedriger als bei den Compomeren, Glasionomerzementen und kunststoffverstärkten Glasionomerzementen.

### Beispiel 5: Lichthärtendes Fissurenversiegelungsmaterial als 1 K-Paste

Aus den nachfolgenden Bestandteilen wurde mit Hilfe eines Laborkneters eine Paste hergestellt.
(A) 21 Gew.-% Di-HEMA-phosphat
(A) 5 Gew.-% Trimellithsäure-di-HEMA-ester
(A) 10 Gew.-% 1,3-Glycerindimethacrylatphosphat
(B) 5 Gew.-% Bis-GMA
(C) 55 Gew.-% Quarz silanisiert mit 3 Gew.-% Methacryloxypropyltrimethoxysilan
(C) 1 Gew.-% Calciumhydroxid
(D) 0,9 Gew.-% Dimethylaminoethylbenzoat
(D) 1,1 Gew.-% Campherchinon
(E) 1 Gew.-% pyrogene Kieselsäure (Aerosil OX 50) silanisiert mit 3 Gew.-% Methacryloxypropyltrimetlioxysilan

| Material | Biegefestigkeit [MPa] | Haftung Dentin [MPa] | Wasseraufnahme [µg/mm³] |
|---|---|---|---|
| Beispiel 5, lc | 50 | 3,1 | 19 |

## Patentansprüche

1. Selbstadhäsives Dentalmaterial, umfassend:
(A) 5 bis 75 Gew.-% einer oder mehrerer mono- oder höherfunktioneller ethylenisch ungesättigter Verbindungen, die zusätzlich über mindestens eine säurefunktionelle Gruppe verfügen, wobei mindestens eine der Verbindungen mindestens eine P-OH-Gruppe aufweist,
(B) 2 bis 50 Gew.-% einer oder mehrerer mono- oder höherfunktioneller ethytenisch ungesättigter Verbindungen ohne säurefunktionelle Gruppe,
(C)22,8 bis 85 Gew.-% Füllstoff(e), umfassend mindestens einen Füllstoff, der mit der Komponente (A) im Sinne einer lonenaustausch-, Neutralisations-, Salzbildungs- und/oder Chelatbildungsreaktion zu reagieren vermag,
(D)0,1 bis 8 Gew.-% eines oder mehrerer Initiatoren und gegebenenfalls Aktivatoren,
(E) 0,1 bis 20 Gew.-% zusätzliche Additive bzw. Modifikatoren,
wobei das Gewichtsverhältnis in % von Komponente (A) zu Komponente (B) im Bereich von 21 bis 90 : 10 bis 79 liegt, und wobei das Wasseraufnahmevermögen bestimmt gemäß EN ISO 4049 : 2000 kleiner 40 µg/mm³ beträgt.

2. Dentalmaterial nach Anspruch 1, wobei das Monomer in der Komponente (A) mit mindestens einer P-OH-Gruppe in einer Konzentration von mindestens 5 Gew.-% vorliegt bezogen auf die Bestandteile (A) bis (E).

3. Dentalmaterial nach einem der vorstehenden Ansprüche, **gekennzeichnet durch** einen Haftwert auf Rinderzahnschmelz oder -dentin von mindestens 2,0 MPa, ohne dass die Zahnhartsubstanz vorbehandelt zu werden braucht, bestimmt gemäß Seite 16, Zeilen 6-26 der Beschreibung in der ursprünglich eingerichten Fassung.

4. Dentalmaterial nach einem der vorstehenden Ansprüche, **gekennzeichnet durch** folgende Eigenschaften:
Haftwert auf Rinderzahnschmelz oder-dentin von mindestens 2,0 MPa, bestimmt gemäß Seite 16, Zeilen 6-26 der Beschreibung in der ursprünglich eingereichten Fassung.
Wasseraufnahmevermögen von < 30 µg / mm³, bestimmt gemäß EN ISO 4049 : 2000
Biegefestigkeit von > 30 MPa bestimmt gemäß EN ISO 4049 : 2000 (3-Punktbiegeversuch).

5. Dentalmaterial nach einem der vorstehenden Ansprüche, wobei es sich bei den polymerisierbaren Gruppierungen der Komponente (A) um Acryl-, Methacryl-, Vinyl- und/oder Styrylgruppen handelt und die Säuregruppen gewählt sind aus Carbonsäureresten, Säureresten des Phosphors, Schwefels und/oder des Bors.

6. Dentalmaterial nach einem der vorstehenden Ansprüche, wobei die Komponente (A) gewählt ist aus: 4-(Meth)acryloyloxyethyltrimellithsäure, Butentricarbonsäure, Bis-4,6- bzw. Bis-2,5-(Meth)acryloyloxyethyltrimellithsäure, Phosphorsäureester von Hydroxyethyl(meth)acrylat (HEMA), Glycerindi(meth)acrylat und/oder Pentaerythrittri(meth)acrylat, Chlor- und Bromphosphorsäureester des Bisphenol-A-Glycidyl(meth)acrylats.

7. Dentalmaterial nach einem der vorstehenden Ansprüche, wobei die Komponente (A) ein Molekulargewicht im Bereich von 70 bis 5000 aufweisen.

8. Dentalmaterial nach einem der vorstehenden Ansprüche, wobei es sich bei den polymerisierbaren Gruppierungen der Komponente (B) um Acryl-, Methacryl-, Vinyl- und/oder Styrylgruppen handelt.

9. Dentalmaterial nach einem der vorstehenden Ansprüche, wobei die Komponente (B) gewählt ist aus: Methyl(meth)acrylat, Butyl(meth)acrylat, 2-Ethylhexyl(meth)acrylat, Lauryl(meth)acrylat, 2-Hydroxyethyl(meth)acrylat, 2-Hydroxypropyl(meth)acrylat, Glycerin-1,3-di(meth)acrylat (GDMA), Glycerin-1,2-di(meth)acrylat, Cyclohexyl(meth)acrylat, Phenyl(meth)acrylat, Isobornyl(meth)-acrylat, Ethylenglykoldi(meth)acrylat, 1,4-Butandioldi(meth)acrylat, Triethylenglykoldi(meth)acrylat (TEGDMA), 1,6-Hexandioldi(meth)acrylat, 1,12-Dodecandioldi(meth)acrylat,Trimethylolpropantri(meth)acrylat, Pentaerythrittetra-(meth)acrylat und Dipentaerythrithexa(meth)acrylat, Bisphenol-A-di(meth)acrylat sowie die davon abgeleiteten ethoxy- bzw. propoxylierten Di(meth)acrylate, Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und / oder Propylenoxideinheiten verlängerten Verbindungen des Bis(hydroxymethyl)-tricyclo[5.2.1.0^{2,6}]-decans, Urethan(meth)acrylate, 7,7,9-Trimethyl-4,13-dioxo-5,12-diazahexadecan-1,16-dioxy-di(meth)acrylat (UDMA).

10. Dentalmaterial nach einem der vorstehenden Ansprüche, wobei der Füllstoff, der mit Komponente (A) im Sinne einer lonenaustausch-, Neutralisations-, Salzbildungs- und/oder Chelatbildungsreaktion zu reagieren vermag, gewählt ist aus: feinverteilten Metallen, Oxiden bzw. Hydroxiden von Calcium, Magnesium, Strontium und Zink, basischen Glaspulvern mit einem hohen Anteil an 2- und 3-wertigen Ionen, Schichtsilikate, Bentonite, Calciumsilikate, Natriumaluminiumsilikate, Zeolithe, Molekularsiebe, Apatit, Borat, Phosphat und Fluoroalumino silikatgläser.

11. Dentalmaterial nach einem der vorstehenden Ansprüche, wobei die Komponente (C) Füllstoffe umfasst, die gegenüber den Säurefunktionen der Komponente (A) inert sind.

12. Dentalmaterial nach einem der vorstehenden Ansprüche, wobei die Füllstoffe, die gegenüber den Säurefunktionen der Komponente (A) inert sind, gewählt sind aus: Quarz, Zirkonsilikate, Fällungskieselsäuren (HDKH) und schwerlösliche Metallsalze wie Bariumsulfat oder Calciumfluorid geeignet.

13. Dentalmaterial nach einem der vorstehenden Ansprüche, wobei die Komponente (D) gewählt ist aus: Photoinitiatoren und/oder Redoxinitiatorsystemen und/oder thermischen Initiatoren.

14. Dentalmaterial nach einem der vorstehenden Ansprüche, wobei die Komponente (D) ein Redoxinitiatorsystem umfasst, umfassend Barbitursäure oder Thiobarbitursäure bzw. ein Barbitursäure- oder Thiobarbitursäurederivat, eine Peroxodisulfatverbindung und/oder eine Peroxodiphosphatverbindung, eine Sulfinsäureverbindung und eine Kupfenrerbindung.

15. Dentalmaterial nach einem der vorstehenden Ansprüche, wobei zur weiteren Beschleunigung der Aushärtung die Polymerisation in Gegenwart von Schwermetallverbindungen durchgeführt wird.

16. Dentalmaterial nach einem der vorstehenden Ansprüche, wobei die Komponente (D) folgende Bestandteile umfasst:
14,9 bis 50 New.-% einer Barbitursäure oder Thiobarbitursäure bzw. einem Barbitursäure- oder Thiobarbitursäurederivat,
30 bis 75 Gew.-% einer Peroxodisulfatverbindung und/oder Peroxodiphosphalverbindung,
10 bis 35 Gew.-% einer Sulfinsäureverbindung und
0,1 bis 5 Gew.-% einer Kupferverbindung.

17. Dentalmaterial nach einem der vorstehenden Ansprüche, wobei die Komponente (E) gewählt ist aus: Weichmachern, anorganische und organische Pigmente bzw. Farbstoffen, Stabilisatoren und/oder Schwermetallfängern, Thixotropiehilfsmitteln, ionenabgebende Substanzen, bakterizid oder antibiotisch wirksamen Substanzen.

18. Selbstadhäsives Dentalmaterial nach einem der Ansprüche 1 bis 8 zur Verwendung als Füllungsmaterial, Fissurenversiegeler, Zement, Stumpfaufbaumaterial oder als zahntechnischer Werkstoff.

19. Verwendung einer Zusammensetzung wie sie in einem der Ansprüche 1 bis 17 beschrieben ist zur Herstellung eines selbstadhäsiven Dentalmaterials zur Anwendung in einem Verfahren, umfassend die Schritte: a) Bereiten einer Präparation in dentalern Hartgewebe, b) direktes Applizieren des selbstadhäsiven Dentalmaterials in die Präparation, c) Härten des selbstadhäsiven Dentalmaterials, wobei vor Schritt b) keine weiteren Behandlungsschritte durchgeführt werden, insbesondere nicht: Konditionieren, Ätzen, Primen, Bonden.

## Claims

1. self-adhesive dental material comprising:
(A) 5% to 75% by weight of one or more monofunctional or polyfunctional ethylenically unsaturated compounds which additionally possess at least one acid-functional group, at least one of the compounds containing at least one P-OH group,
(B) 2% to 50% by weight of one or more monofunctional or polyfunctional ethylenically unsaturated compounds without an acid-functional group,
(C) 22.8% to 85% by weight of filler(s), including at least one filler capable of reacting with component (A) in an ion-exchange, neutralization, salt-forming and/or chelate-forming reaction,
(D) 0.1% to 8% by weight of one or more initiators and, if desired, activators,
(E) 0.1% to 20% by weight of additional additives and/or modifiers,
the weight ratio in % of components (A) to (B) being in the range from 21 to 90:10 to 79, and the water absorbency as determined in accordance with EN ISO 4049:2000 being less than 40 µg/mm³.

2. Dental material according to claim 1, wherein the monomer in component (A) having at least one P-OH group is present in a concentration of at least 5% by weight based on the constituents (A) to (E).

3. Dental material according to any of the preceding claims, **characterized by** an adhesion value to bovine dental enamel or bovine dentine of at least 2.0 MPa without any need to pretreat the dental hard substance, as determined in accordance with page 16 lines 6-26 of the description as filed.

4. Dental material according to any of the preceding claims, **characterized by** the following properties:
adhesion value to bovine dental enamel or bovine dentine of at least 2.0 MPa, as determined in accordance with page 16 lines 6-26 of the description as filed,
water absorbency of < 30 µg/mm³, as determined in accordance with EN ISO 4049:2000,
flexural strength of > 30 MPa, as determined in accordance with EN ISO 4049:2000 (3-point bending test).

5. Dental material according to any of the preceding claims, wherein the polymerizable groups of component (A) comprise acrylic, methacrylic, vinyl and/or styryl groups and the acid groups are selected from carboxylic acid residues, acid residues of phosphorus, of sulphur and/or of boron.

6. Dental material according to any of the preceding claims, wherein component (A) is selected from: 4-(meth)acryloyloxyethyltrimellitic acid, butenetricarboxylic acid, bis-4,6- and/or bis-2,5-(meth)acryloyloxyethyltrimellitic acid, phosphoric esters of hydroxyethyl (meth)acrylate (HEMA), glycerol di(meth)acrylate and/or pentaerythritol tri(meth)acrylate, chloro- and bromophosphoric esters of bisphenol A-glycidyl (meth) acrylate.

7. Dental material according to any of the preceding claims, wherein component (A) has a molecular weight in the range from 70 to 5000.

8. Dental material according to any of the preceding claims, wherein the polymerizable groups of component (B) are acrylic, methacrylic, vinyl and/or styryl groups.

9. Dental material according to any of the preceding claims, wherein component (B) is selected from: methyl (meth)acrylate, butyl (meth)acrylate, 2-ethylhexyl (meth)acrylate, lauryl (meth)acrylate, 2-hydroxyethyl (meth)acrylate, 2-hydroxypropyl (meth)acrylate, glycerol 1,3-di(meth)acrylate (GDMA), glycerol 1,2-di(meth)acrylate, cyclohexyl (meth)acrylate, phenyl (meth)acrylate, isobornyl (meth)acrylate, ethylene glycol di(meth)acrylate, 1,4-butanediol di(meth)acrylate, triethylene glycol di(meth)acrylate (TEGDMA), 1,6-hexanediol di(meth)acrylate, 1,12-dodecanediol di(meth)acrylate, trimethylolpropane tri(meth)acrylate, pentaerythritol tetra(meth)acrylate and dipentaerythritol hexa(meth)acrylate, bisphenol A di(meth)acrylate and also the ethoxylated and/or propoxylated di(meth)acrylates derived therefrom, diacrylic and dimethacrylic esters of bis(hydroxymethyl)tricyclo[5.2.1.0^{2,6}]decane, and the diacrylic and dimethacrylic esters of compounds of bis(hydroxymethyl)tricycle[5.2.1.0^{2,6}]decane that have been extended with 1 to 3 ethylene oxide and/or propylene oxide unites, urethane (meth)acrylates and 7,7,9-trimethyl-4,13-dioxo-5,12-diazahexadecane-1,16-dioxy di(meth)acrylate (UDMA).

10. Dental material according to any of the preceding claims, wherein the filler capable of reacting with component (A) in an ion-exchange, neutralization, salt-forming and/or chelate-forming reaction is selected from: finely divided metals, oxides and/or hydroxides of calcium, magnesium, strontium and zinc, basic glass powders with a high fraction of 2- and 3-valent ions, phyllosilicates, bentonites, calcium silicates, sodium aluminium silicates, zeolites, molecular sieves, apatite, and borate, phosphate and fluoroaluminosilicate glasses.

11. Dental material according to any of the preceding claims, wherein component (C) comprises fillers which are inert toward the acid functions of component (A).

12. Dental material according to any of the preceding claims, wherein the fillers which are inert toward the acid functions of component (A) are selected from: quartz, zirconium silicates, precipitated silicas (HDKH) and low-solubility metal salts such as barium sulphate or calcium fluoride.

13. Dental material according to any of the preceding claims, wherein component (D) is selected from: photoinitiators and/or redox initiator systems and/or thermal initiators.

14. Dental material according to any of the preceding claims, wherein component (D) comprises a redox initiator system comprising barbituric acid or thiobarbituric acid and/or a barbituric or thiobarbituric acid derivative, a peroxodisulphate compound and/or a peroxodiphosphate compound, a sulphinic acid compound and a copper compound.

15. Dental material according to any of the preceding claims, wherein curing is further accelerated by carrying out the polymerization in the presence of heavy metal compounds.

16. Dental material according to any of the preceding claims, wherein component (D) comprises the following constituents:
14.9% to 50% by weight of a barbituric acid or thiobarbituric acid and/or a barbituric or thiobarbituric acid derivative,
30% to 75% by weight of a peroxodisulphate compound and/or peroxodiphosphate compound,
10% to 35% by weight of a sulphinic acid compound and
0.1% to 5% by weight of a copper compound.

17. Dental material according to any of the preceding claims, wherein component (E) is selected from: plasticizers, organic and inorganic pigments and/or dyes, stabilizers and/or heavy metal scavengers, thixotropic assistants, ion donor substances, bactericidal or antibiotic substances.

18. Self-adhesive dental material according to any of claims 1 to 8 for use as filling material, fissure sealant, cement, core build-up material or as dental engineering material.

19. Use of a composition as described in any of claims 1 to 17 to produce a self-adhesive dental material for use in a process comprising the following steps: a) carrying out a preparation in hard dental tissue, b) directly applying the self-adhesive dental material into the preparation, c) curing the self-adhesive dental material, in which no further treatment steps are carried out before step b), especially not the following: conditioning, etching, priming, bonding

## Revendications

1. Matériau dentaire autoadhésif, comprenant :
(A) 5 à 75 % en poids d'un ou plusieurs composés à insaturation éthylénique, mono- ou polyfonctionnels, qui en outre sont dotés d'au moins un groupe à fonction acide, au moins l'un des composés comportant au moins un groupe P-OH,
(B) 2 à 50 % en poids d'un ou plusieurs composés à insaturation éthylénique, mono- ou polyfonctionnels, dépourvus de groupe à fonction acide,
(C) 22,8 à 85 % en poids de charge(s), comprenant au moins une charge qui peut réagir avec le composant (A) dans le sens d'une réaction d'échange d'ions, de neutralisation, de salification et/ou de formation de chélate,
(D) 0,1 à 8 % en poids d'un ou plusieurs amorceurs et éventuellement activateurs,
(E) 0,1 à 20 % en poids de modificateurs ou d'additifs supplémentaires,
le rapport pondéral en % du composant (A) au composant (B) se situant dans la plage allant de 21 à 90 : 10 à 79 et le pouvoir d'absorption d'eau, déterminé selon la norme EN ISO 4049 : 2000, étant inférieur à 40 µg/mm³.

2. Matériau dentaire selon la revendication 1, dans lequel le monomère dans le composant (A) comportant au moins un groupe P-OH est présent à une concentration d'au moins 5 % en poids, par rapport aux composants (A) à (E).

3. Matériau dentaire selon l'une quelconque des revendications précédentes, **caractérisé par** un coefficient d'adhérence à la dentine ou à l'émail de dent bovine, déterminé comme indiqué page 16, lignes 6-29 de la description dans la version déposée initialement, d'au moins 2,0 MPa, sans que le tissu dur de la dent ait besoin d'être prétraité.

4. Matériau dentaire selon l'une quelconque des revendications précédentes, **caractérisé par** les propriétés suivantes :
coefficient d'adhérence à la dentine ou à l'émail de dent bovine, déterminé comme indiqué page 16, lignes 6-29 de la description dans la version déposée initialement, d'au moins 2,0 MPa,
pouvoir d'absorption d'eau, déterminé selon la norme EN ISO 4049 : 2000, de < 30 µg/mm³,
résistance à la flexion, déterminée selon la norme EN ISO 4049 : 2000 (essai en flexion en trois pointes), de > 30 MPa.

5. Matériau dentaire selon l'une quelconque des revendications précédentes, dans lequel les groupements polymérisables du composant (A) consistent en groupes acrylique, méthacrylique, vinyle et/ou styryle et les groupes acides sont choisis parmi des restes d'acides carboxyliques, des restes acides du phosphore, du soufre ou du bore.

6. Matériau dentaire selon l'une quelconque des revendications précédentes, dans lequel le composant (A) est choisi parmi : l'acide 4-(méth)-acryloyloxyéthyltrimellitique, l'acide butène-tricarboxylique, l'acide bis-4,6- ou bis-2,5-(méth)acryloyloxyéthyltrimellitique, les phosphates de (méth)acrylate d'hydroxyéthyle (HEMA), di(méth)acrylate de glycérol et/ou tri(tnéth)acrylate de pentaérythritol, les chlore- et bromophosphates du glycidyl(méth)acrylate de bisphénol A.

7. Matériau dentaire selon l'une quelconque des revendications précédentes, dans lequel le composant (A) présente une masse moléculaire dans la plage de 70 à 5 000.

8. Matériau dentaire selon l'une quelconque des revendications précédentes, dans lequel les groupements polymérisables du composant (B) consistent en des groupes acrylique, méthacrylique, vinyle et/ou styryle.

9. Matériau dentaire selon l'une quelconque des revendications précédentes, dans lequel le composant (B) est choisi parmi : le (méth)acrylate de méthyle, le (méth)acrylate de butyle, le (méth)acrylate de 2-éthylhexyle, le (méth)acrylate de lauryle, le (méth)acrylate de 2-hydroxyéthyle, le (méth)acrylate de 2-hydroxypropyle, le 1,3-di(méth)acrylate de glycérol (GDMA), le 1,2-di(méth)acrylate de glycérol, le (méth)acrylate de cyclohexyle, le (méth)acrylate de phényle, le (méth)acrylate d'isobornyle, le di(méth)acrylate d'éthylèneglycol, le di(méth)acrylate de 1,4-butanediol, le di(méth)acrylate de triéthylèneglycol (TEGDMA), le di(méth)acrylate de 1,6-hexanediol, le di(méth)acrylate de 1,12-dodécanediol, le tri(méth)acrylate de triméthylolpropane, le tétra(méth)acrylate de pentaérythritol et l'hexa(méth)acrylate de dipentaérythritol, le di(méth)acrylate de bisphénol A ainsi que les di(méth)acrylates éthoxylés ou propoxylés dérivés de ceux-ci, les diacrylates et diméthacrylates du bis(hydroxyméthyl)-tricyclo[5.2.10^{2.6}]-décane et les diacrylates et diméthacrylates des composés, prolongés par 1 à 3 groupes oxyde d'éthylène et/ou oxyde de propylène du bis(hydroxyméthyl)-tricyclo[5.2.1.0^{2.6}]-décane, les uréthanne-(méth)acrylates, le 7,7,9-triméthyl-4,13-dioxo-5,12-diazahexadécane-1,16-dioxy-di(méth)acrylate (UDMA).

10. Matériau dentaire selon l'une quelconque des revendications précédentes, dans lequel la charge qui peut réagir avec le composant (A) dans le sens d'une réaction d'échange d'ions, de neutralisation, de salification et/ou de formation de chélate est choisi parmi des métaux finement divisés, des oxydes ou hydroxydes de calcium, magnésium, strontium et zinc, des poudres de verre basiques ayant une forte teneur en ions di- et trivalents, les silicates lamellaires, les bentonites, les silicates de calcium, les aluminosilicates de sodium, les zéolithes, les tamis moléculaires, l'apatite, les verres de borate-, phosphate- et fluoroaluminosilicate.

11. Matériau dentaire selon l'une quelconque des revendications précédentes, dans lequel le composant (C) comprend des charges qui sont inertes vis-à-vis des fonctions acides du composant (A).

12. Matériau dentaire selon l'une quelconque des revendications précédentes, dans lequel les charges qui sont inertes vis-à-vis des fonctions acides du composant (A) sont choisies parmi le quartz, les silicates de zirconium, les acides siliques précipités (NDKH) et des sels métalliques peu solubles tels que le sulfate de baryum ou le fluorure de calcium.

13. Matériau dentaire selon l'une quelconque des revendications précédentes, dans lequel le composant (D) est choisi parmi : des photoamorceurs et/ou des systèmes amorceurs redox et/ou des amorceurs thermiques.

14. Matériau dentaire selon l'une quelconque des revendications précédentes, dans lequel le composant (D) comprend un système amorceur redox, comprenant l'acide barbiturique ou l'acide thiobarbiturique ou un dérivé d'acide barbiturique ou d'acide thiobarbiturique, un composé peroxodisulfate et/ou un composé peroxodiphosphate, un composé acide sulfinique et un composé contenant du cuivre.

15. Matériau dentaire selon l'une quelconque des revendications précédentes, dans lequel, pour accélérer encore le durcissement, on effectue la polymérisation en présence de composés contenant des métaux lourds.

16. Matériau dentaire selon l'une quelconque des revendications précédentes, dans lequel le composant (D) comprend les composants suivants :
14,9 à 50 % en poids d'un acide barbiturique ou thiobarbiturique ou d'un dérivé d'acide barbiturique ou thiobarbiturique,
30 à 75 % % en poids d'un composé peroxodisulfate et/ou d'un composé peroxodiphosphate,
10 à 35 % en poids d'un composé acide sulfinique et
0,1 à 5 % en poids d'un composé contenant du cuivre.

17. Matériau dentaire selon l'une quelconque des revendications précédentes, dans lequel le composant (E) est choisi parmi : des plastifiants, des colorants ou pigments organiques ou inorganiques, des stabilisants et/ou des capteurs de métaux lourds, des adjuvants de thixotropie, des substances libérant des ions, des substances à activité bactéricide ou antibiotique.

18. Matériau autoadhésif selon l'une quelconque des revendications 1 à 8, pour utilisation en tant que matériau de plombage, bouche-fissures, ciment, matériau de construction sur moignon ou en tant que matériau de prothèse dentaire.

19. Utilisation d'une composition telle que décrite dans l'une quelconque des revendications 1 à 17, pour la production d'un matériau dentaire autoadhésif destiné à être utilisé dans un procédé comprenant les étapes : a) production d'une préparation en tissu dentaire dur, b) application directe du matériau autoadhésif dans la préparation, c) durcissement du matériau dentaire autoadhésif, aucune autre étape de traitement n'étant effectuée avant l'étape b), en particulier aucun conditionnement, aucune attaque, aucun apprêtage, aucune enduction.
